# EUROPÄISCHE PATENTSCHRIFT

(11) **EP 2 429 492 B1**
(45) Veröffentlichungstag und Bekanntmachung des Hinweises auf die Patenterteilung: **25.06.2014**
(21) Anmeldenummer: 10718209.9
(22) Anmeldetag: 11.05.2010
(51) Int. Cl.: A61K 9/20, A61K 9/14, A61K 47/10, A61K 47/34, A61K 31/00

(54) **Feste pharmazeutische Zubereitungen enthaltend Copolymere auf Basis von Polyethern in Kombination mit wasserschwerlöslichen Polymeren**
Solid pharmaceutical preparations containing copolymers based on polyethers combined with poorly water-soluble polymers
Préparations pharmaceutiques solides contenant des copolymères à base de polyéthers en association avec des polymères peu solubles dans l'eau

(30) Priorität: 13.05.2009 EP 09160129
(43) Veröffentlichungstag der Anmeldung: 21.03.2012
(73) Patentinhaber: BASF SE, 67056 Ludwigshafen (DE)
(72) Erfinder: KOLTER, Karl, 67117 Limburgerhof (DE); DJURIC, Dejan, 68165 Mannheim (DE); FISCHER, Stefan, 67251 Freinsheim (DE)
(86) Internationale Anmeldenummer: PCT/EP2010/056447
(87) Internationale Veröffentlichungsnummer: WO 2010/130728

(56) Entgegenhaltungen:
- WO-A1-2009/013202
- WO-A2-2007/051743
- US-A1- 2007 148 232
- LEUNER C ET AL: "Improving drug solubility for oral delivery using solid dispersions" EUROPEAN JOURNAL OF PHARMACEUTICS AND BIOPHARMACEUTICS, ELSEVIER SCIENCE PUBLISHERS B.V., AMSTERDAM, NL, Bd. 50, Nr. 1, 3. Juli 2000 (2000-07-03), Seiten 47-60, XP004257179 ISSN: 0939-6411 DOI: DOI:10.1016/S0939-6411(00)00076-X
- DATABASE EMBASE [Online] ELSEVIER SCIENCE PUBLISHERS, AMSTERDAM, NL; März 2009 (2009-03), YUAN J ET AL: "Influence of solid dispersion technique combination on dissolution of tanshinone IIA" XP002613412 Database accession no. EMB-2009298320 & ZHONGGUO ZHONGYAO ZAZHI 2009 ZHONGGUO ZHONGYI YANJIUYUAN CHN, Bd. 34, Nr. 6, März 2009 (2009-03), Seiten 685-689, ISSN: 1001-5302

## Beschreibung

Die vorliegende Erfindung betrifft feste pharmazeutische Zubereitungen von Polyether-Copolymeren, die durch Polymerisation von Vinylacetat und N-Vinyllactamen in Gegenwart eines Polyethers erhalten werden, und in Wasser schwerlöslichen Wirkstoffen, in Kombination mit weiteren Polymeren, die in der Lage sind, die Stabilität der Formulierung und/oder die Freisetzung der biologisch aktiven Substanz zu beeinflussen. Weiterhin betrifft die Erfindung Verfahren zur Herstellung dieser Zubereitungen sowie deren Verwendung.

Die entsprechenden Polyether-Copolymere fungieren als Solubilisatoren für die in Wasser schwerlöslichen biologisch aktiven Substanzen.

Bei der Herstellung homogener Zubereitungen insbesondere von biologisch aktiven Substanzen hat die Solubilisierung von hydrophoben, also in Wasser schwerlöslichen Stoffen, eine sehr große praktische Bedeutung erlangt.

Unter Solubilisierung ist das Löslichmachen von in einem bestimmtem Lösungsmittel, insbesondere Wasser, schwer- oder unlöslichen Substanzen durch grenzflächenaktive Verbindungen, die Solubilisatoren, zu verstehen. Solche Solubilisatoren sind in der Lage, schlecht wasserlösliche oder wasserunlösliche Stoffe in klare, höchstens opaleszierende wässrige Lösungen zu überführen, ohne dass hierbei die chemische Struktur dieser Stoffe eine Veränderung erfährt (Vgl. Römpp Chemie Lexikon, 9. Auflage, Bd.5. S. 4203, Thieme Verlag, Stuttgart, 1992).

Die hergestellten Solubilisate sind dadurch gekennzeichnet, dass der schlecht wasserlösliche oder wasserunlösliche Stoff in den Molekülassoziaten der oberflächenaktiven Verbindungen, die sich in wässriger Lösung bilden, wie beispielsweise hydrophobe Domänen oder Mizellen, kolloidal gelöst vorliegt. Die resultierenden Lösungen sind stabile oder metastabile einphasige Systeme, die optisch klar bis opaleszent erscheinen.

Im Falle von pharmazeutischen Zubereitungen kann die Bioverfügbarkeit und damit die Wirkung von Arzneistoffen durch die Verwendung von Solubilisatoren gesteigert werden.

Eine weitere wünschenswerte Anforderung an Solubilisatoren ist die Fähigkeit, mit schwerlöslichen Substanzen sogenannte "feste Lösungen" auszubilden. Der Begriff "feste Lösung" bezeichnet einen Zustand, in dem eine Substanz kolloiddispers oder im Idealfall molekulardispers in einer festen Matrix, beispielsweise einer Polymermatrix, verteilt ist. Solche festen Lösungen führen zum Beispiel bei Verwendung in festen pharmazeutischen Darreichungsformen eines schwerlöslichen Wirkstoffs zu einer verbesserten Freisetzung des Wirkstoffs. Eine wichtige Anforderung an solche feste Lösungen ist, dass sie auch bei Lagerung über längere Zeit stabil sind, d.h., dass der Wirkstoff nicht auskristallisiert. Weiterhin ist auch die Kapazität der festen Lösung, mit anderen Worten die Fähigkeit der Ausbildung von stabilen festen Lösungen mit möglichst hohen Wirkstoffgehalten, von Bedeutung.

Bei der Ausbildung von festen Lösungen spielt neben der grundsätzlichen Fähigkeit der Solubilisatoren zur Bildung von festen Lösungen auch die Hygroskopizität der Solubilisatoren eine bedeutende Rolle. Solubilisatoren, die aus der Umgebungsluft zuviel Wasser aufnehmen, führen zu einem Zerfließen der festen Lösung und der unerwünschten Kristallisation der Wirkstoffe. Auch bei der Verarbeitung zu Darreichungsformen kann eine zu große Hygroskopizität Probleme bereiten. ------

Viele bekannte polymere Solubilisatoren weisen die Nachteile auf, dass sie keine stabilen festen Lösungen ausbilden, insbesondere dann wenn der Wirkstoff oberhalb der Sättigungskonzentration im Polymer vorliegt. Hierbei ist das System kinetisch kontrolliert und der Wirkstoff kann im Laufe der Lagerung auskristallisieren. Dies stellt ein großes Problem dar.

Außerdem lassen die bekannten Solubilisatoren und Formulierungen noch Raum für Verbesserungen, was die Solubilisierung in wässrigen Systemen insbesondere biologischen Systemen betrifft. Die orale Bioverfügbarkeit ist häufig nicht in dem Maße gesteigert wie es wünschenswert wäre, um eine gleichmäßige, reproduzierbare Wirkung ohne Nebenwirkungen zu erzielen.

Auch hinsichtlich der Verarbeitbarkeit weisen einige der bekannten Solubilisatoren aufgrund ihrer Neigung zu Klebrigkeit Nachteile auf, da sie keine ausreichend fließfähigen Pulver darstellen.

Ein weiteres Problem bei pharmazeutischen Zubereitungen von in Wasser schwerlöslichen Wirkstoffen ist die Steuerung der Freisetzung. Häufig übt bei solchen Formen nicht der Hilfsstoff die Steuerung der Freisetzung aus, sondern die Kristalleigenschaften des Wirkstoffes. Das bedeutet, dass Schwankungen in der Teilchengröße des Wirkstoffes, Unterschiede in der Kristallmodifikation und Unterschiede in der Kornform die Auflösung erheblich beeinflussen. Da diese Größen schwierig exakt einzustellen sind, resultieren unterschiedliche Freisetzungsraten. Bei wasserlöslichen Wirkstoffen ist dieses Problem nicht vorhanden, da der Auflösungsprozess des Wirkstoffes erheblich schneller ist als der Diffusionsprozess, der durch ein Retardpolymer gesteuert wird. Im Hinblick auf die Freisetzung ist zu unterscheiden zwischen Instant Release (schnellfreisetzenden) Formen, Enteric Release(gastric resistant, magensaftresistenten) Formen und Sustained Release (retardiert, langsam freisetzenden) Formen. Als Instant Release Formen werden Formen bezeichnet, die mindestens 75% nach 1 h freisetzen. Bei Enteric Release Formen erfolgt eine geringe Freisetzung in Magensaft (< 10% in der Regel), aber eine schnelle in Darmsaft. Sustained Release Formen weisen sowohl in Magensaft als auch in Darmsaft eine langsame Freisetzung auf. Von Sustained Release Formen spricht man, wenn die Freisetzung langsamer ist als 75% nach 3h.

Unter den in Wasser schwerlöslichen Polymeren sind Polymere zu verstehen, die über den ganzen pH-Bereich oder zumindest in einem gewissen pH-Bereich schwerlöslich sind. Hierunter fallen sogenannte Retardpolymere (unlöslich in der Regel von pH 1 - 14), saure, magensaftresistente Polymere (schwerlöslich im sauren pH-Bereich) und basische, reverse enteric Polymere (schwerlöslich im neutralen bis basischen pH-Bereich). Zur Definition von Löslichkeit siehe weiter unten.

In der EP-A 876 819 ist die Verwendung von Copolymeren aus mindestens 60 Gew.-% N-Vinylpyrrolidon und Amiden oder Estern mit langkettigen Alkylgruppen beschrieben.

In der EP-A 948 957 ist die Verwendung von Copolymerisaten aus monoethylenisch ungesättigten Carbonsäuren wie beispielsweise Acrylsäure und hydrophob modifizierten Comonomeren wie beispielsweise N-Alkyl- oder N,N-Dialkyl- Amiden ungesättigter Carbonsäuren mit C₈-C₃₀-Alkylresten beschrieben.

Aus der DE-A 199 350 63 sind Polyalkylenoxid-haltige Pfropfpolymere auf Basis von Vinyllactamen und Vinylacetat sowie deren Verwendung als Gashydratinhibitoren bekannt.

Aus der EP-A 953 347 ist die Verwendung von Polyalkylenoxid-haltigen Pfropfpolymerisaten als Solubilisatoren bekannt. Die dort beschriebenen Pfropfpolymerisate aus Vinylacetat und Polyalkylenoxiden stellen häufig keine Pulver sondern zähklebrige Flüssigkeiten dar, was anwendungstechnisch von Nachteil ist.

Aus der WO 2007/051743 ist die Verwendung von wasserlöslichen oder wasserdispergierbaren Copolymerisatenaus N-Vinyllactam, Vinylacetat und Polyethern , als Solubilisatoren für pharmazeutische, kosmetische, lebensmitteltechnische, agrotechnische oder sonstige technische Anwendungen bekannt. Darin wird ganz allgemein beschrieben, dass die entsprechende Pfropfpolymerisate auch in der Schmelze mit den Wirkstoffen verarbeitet werden können.

Aus der WO 2009/013202 ist bekannt, dass solche Pfropfpolymerisate aus N-Vinyllactam, Vinylacetat und Polyethern im Extruder aufgeschmolzen und mit pulverförmigen oder flüssigen Wirkstoffen vermischt werden können, wobei die Extrusion bei Temperaturen deutlich unter dem Schmelzpunkt des Wirkstoffs beschrieben ist..

Allerdings lässt sich durch Vermischen des geschmolzenen Pfropfpolymeren mit pulverförmigen oder flüssigen Wirkstoffen keine befriedigend hohe und gleichzeitig stabile Wirkstoffbeladung erzielen. Vor allem die Erreichung eines stabilen röntgenamorphen Zustands des Wirkstoffs gelingt nicht immer in befriedigendem Maße.

Aufgabe der vorliegenden Erfindung war es, verbesserte Zubereitungen schwerwasserlöslicher Wirkstoffe zu finden, die bei guter Bioverfügbarkeit eine gezielte Einstellung der Freisetzung ermöglichen.

Demgemäß wurden pharmazeutische Dosierungsformen, enthaltend Zubereitungen von in Wasser schwerlösliche Wirkstoffen in einer Polymermatrix aus PolyetherCopolymeren, wobei die Polyether-Copolymere erhalten werden durch radikalisch initiierte Polymerisation einer Mischung aus 30 bis 80 Gew.-% N-Vinyllactam,10 bis 50 Gew.-% Vinylacetat und 10 bis 50 Gew.-% eines Polyethers, und aus mindestens einem in Wasser schwerlöslichen Polymeren, in denen der in Wasser schwerlösliche Wirkstoff amorph in der Polymermatrix vorliegt, gefunden.

Weiterhin wurde ein Verfahren zur Herstellung von Zubereitungen von in Wasser schwerlöslichen Wirkstoffen gefunden , wobei die Wirkstoffe amorph in einer Polymermatrix auf Basis von Polyether-Copolymeren aus 30 bis 80 Gew.-% N-Vinyllactam,10 bis 50 Gew.-% Vinylacetat und 10 bis 50 Gew.-% eines Polyethers eingebettet vorliegen , welches dadurch gekennzeichnet ist, dass in die Polymermatrix neben dem Polyether-Copolymeren mindestens ein in Wasser schwerlöslichen Polymer eingearbeitet wird und die Polymere mit den in Wasser schwerlöslichen Wirkstoffen innig vermischt werden.

Die in der Polymermatrix vorliegenden Polyether-Copolymeren, werden erhalten durch radikalisch initiierte Polymerisation einer Mischung aus
i) 30 bis 80 Gew.-% N-Vinyllactam,
ii) 10 bis 50 Gew.-% Vinylacetat und
iii) 10 bis 50 Gew.-% eines Polyethers,
mit der Maßgabe, dass die Summe von i), ii) und iii) gleich 100 Gew.-% ist.

Gemäß einer Verfahrensvariante werden die Polyether-Copolymere mit in Wasser schwerlöslichen Polymeren und den in Wasser schwerlöslichen Wirkstoffen innig vermischt und die Mischung über die Glasübergangstemperatur der Copolymeren erwärmt.

Gemäß einer weiteren Verfahrensvariante wird die Mischung aus Polymeren und Wirkstoffen in organischer Lösung hergestellt und anschliessend getrocknet.

Die Polyether-Copolymere sind in Wasser leicht löslich, was bedeutet, dass sich bei 20 °C 1 Teil Copolymer in 1 bis 10 Teilen Wasser löst.

Gemäß einer Ausführungsform der Erfindung werden bevorzugte Polyether-Copolymere, erhalten aus:
i) 30 bis 70 Gew.-% N-Vinyllactam
ii) 15 bis 35 Gew.-% Vinylacetat, und
iii) 10 bis 35 Gew.-% eines Polyethers, verwendet.

Besonders bevorzugt verwendete Polyether-Copolymere sind erhältlich aus:
i) 40 bis 60 Gew.-% N-Vinyllactam
ii) 15 bis 35 Gew.-% Vinylacetat
iii) 10 bis 30 Gew.-% eines Polyethers

Ganz besonders bevorzugt verwendete Polyether-Copolymere sind erhältlich aus
i) 50 bis 60 Gew.-% N-Vinyllactam
ii) 25 bis 35 Gew.-% Vinylacetat, und
iii) 10 bis 20 Gew.-% eines Polyethers,

Auch für die bevorzugten und besonders bevorzugten Zusammensetzungen gilt die Maßgabe, dass die Summe der Komponenten i), ii), und iii) gleich 100 Gew.-% beträgt.

Als N-Vinyllactam kommen N-Vinylcaprolactam oder N-Vinylpyrrolidon oder deren Mischungen in Betracht. Bevorzugt wird N-Vinylcaprolactam verwendet.

Als Pfropfgrundlage dienen Polyether. Als Polyether kommen vorzugsweise Polyalkylenglykole in Betracht. Die Polyalkylenglykole können Molekulargewichte von 1000 bis 100000 D [Dalton], vorzugsweise 1500 bis 35000 D, besonders bevorzugt 1500 bis 10000 D, aufweisen. Die Molekulargewichte werden ausgehend von der gemäß DIN 53240 gemessenen OH-Zahl bestimmt.

Als besonders bevorzugte Polyalkylenglykole kommen Polyethylenglykole in Betracht. Weiterhin eignen sich auch Polypropylenglykole, Polytetrahydrofurane oder Polybutylenglykole, die aus 2-Ethyloxiran oder 2,3-Dimethyloxiran erhalten werden.

Geeignete Polyether sind auch statistische oder blockartige Copolymere von aus Ethylenoxid, Propylenoxid und Butylenoxiden gewonnenen Polyalkylenglykolen wie beispielsweise Polyethylenglykol-Polypropylenglykol-Blockcopolymere. Die Blockcopolymere können vom AB- oder vom ABA-Typ sein.

Zu den bevorzugten Polyalkylenglykolen gehören auch solche, die an einer oder an beiden OH-Endgruppen alkyliert sind. Als Alkylreste kommen verzeigte oder unverzweigte C₁- bis C₂₂-Alkylreste in Betracht, bevorzugt C₁-C₁₈-Alkylreste, beispielsweise Methyl-, Ethyl-, n-Butyl-, Isobutyl-, Pentyl-, Hexyl-, Octyl-, Nonyl-, Decyl-, Dodecyl-, Tridecyl- oder Octadecyl-Reste.

Allgemeine Verfahren zur Herstellung der erfindungsgemäßen Polyether-Copolymere sind an sich bekannt. Die Herstellung erfolgt durch radikalisch initiierte Polymerisation, bevorzugt in Lösung, in nichtwässrigen, organischen Lösungsmitteln oder in gemischt nichtwässrigen/wässrigen Lösungsmitteln. Geeignete Herstellverfahren sind beispielsweise in der WO 2007/051743 und der WO 2009/013202 beschrieben, auf deren Offenbarung hinsichtlich des Herstellungsverfahrens ausdrücklich Bezug genommen wird.

Zur gezielten Steuerung der Freisetzung werden die solubilisierend wirkenden Copolymeren in Kombination mit in Wasser schwerlöslichen Polymeren, die geeignet sind, die Freisetzung der biologisch aktiven Substanz zu beeinflussen, verwendet. Das Ausmaß der Freisetzungsbeeinflussung hängt hierbei in der Regel von der Konzentration des in Wasser schwerlöslichen Polymers ab.

Das Verhältnis des Polyether-Copolymers zu dem in Wasser schwerlöslichen Polymer kann zwischen 99 :1 und 10 : 90 betragen. Vorzugsweise liegt es zwischen 90 : 10 und 30 : 70 und besonders bevorzugt zwischen 80 : 20 und 40 : 60.

Bezüglich der Löslichkeit der Polymere ist der Begriff "in Wasser schwerlöslich" wie folgt zu verstehen: Der Begriff "in Wasser schwerlöslich" umfasst erfindungsgemäß schwerlösliche sowie auch praktisch unlösliche Substanzen und bedeutet, dass für eine Lösung des Polymers in Wasser bei 20 °C mindestens 100 bis 1000 g Wasser pro g Polymer benötigt wird. Bei praktisch unlöslichen Substanzen werden mindestens 10.000 g Wasser pro g Substanz benötigt.

In der folgenden Beschreibung der in Wasser schwerlöslichen Polymere wird der Begriff "schwerlöslich" verkürzt für "in Wasser schwerlöslich" benutzt

### In Wasser schwerlösliche Polymere

In Wasser schwerlösliche Polymere im Sinne der Erfindung sind entweder neutrale schwerlösliche Polymere (Retardpolymere), anionische schwerlösliche Polymere (magensaftresistente Polymere) oder basische schwerlösliche Polymere

### Neutrale schwerlösliche Polymere

Unter schwerlöslichen Polymeren werden solche Polymere verstanden, die über den gesamten pH-Bereich von 1 bis 14 wasserschwerlöslich bzw. lediglich in Wasser quellbar sind. In der Regel ist in der pharmazeutischen Zusammensetzung nur ein wasserunlösliches Polymer enthalten. Es können jedoch gegebenenfalls auch zwei oder mehr wasserunlösliche Polymere nebeneinander oder in Mischung vorliegen.

### Geeignete schwerlösliche Polymere sind beispielsweise:

Neutrale oder im wesentlichen neutrale Methacrylat-Copolymere. Diese können insbesondere aus mindestens 95, insbesondere mindestens 98, bevorzugt mindestens 99, insbesondere zu mindestens 99, besonders bevorzugt zu 100 Gew.-% aus radikalisch polymerisierten (Meth)acrylat-Monomeren mit neutralen Resten, insbesondere C1- bis C4-Alkylresten, bestehen.

Geeignete (Meth)acrylat-Monomere mit neutralen Resten sind z. B. Methylmethacrylat, Ethylmethacrylat, Butylmethacrylat, Methylacrylat, Ethylacrylat, Butylacrylat. Bevorzugt sind Methylmethacrylat, Ethylacrylat und Methylacrylat.

In geringen Anteilen, zu weniger als 5, bevorzugt höchstens 2, besonders bevorzugt höchstens 1 oder 0,05 bis 1 Gew.-% können Methacrylatmonomere mit anionischen Resten, z. B. Acrylsäure und/oder Methacrylsäure, enthalten sein.

Geeignet sind z. B. neutrale oder nahezu neutrale (Meth)acrylat Copolymere aus 20 bis 40 Gew.-% Ethylacrylat, 60 bis 80 Gew.-% Methylmethacrylat und 0 bis weniger als 5, bevorzugt 0 bis 2 oder 0,05 bis 1 Gew.-% (Typ Eudragit® NE).

Eudragit NE ist ein Copolymer aus 30 Gew.-% Ethylacrylat und 70 Gew.-% Methylmethacrylat.

Weitere geeignete schwerlösliche (Meth)acrylat-Copolymere sind beispielsweise unabhängig vom pH-Wert lösliche oder quellbare Polymerisate, die für Arzneimittelüberzüge geeignet sind.

Das schwerlösliche Polymer kann ein Polymerisat aus 98 bis 85 Gew.-% C1- bis C4-Al kylestern der Acryl- oder der Methacrylsäure und 2 bis 15 Gew.-% (Meth)acrylat Monomeren mit einer quaternären Ammoniumgruppe ist oder eine Mischung mehrerer Polymer dieser Substanzklasse sein.

Das schwerlösliche Polymer kann auch ein Polymerisat aus 97 bis mehr als 93 Gew.-% C1- bis C4-Alkylestern der Acryl- oder der Methacrylsäure und 3 bisweniger als 7 Gew.-% (Meth)acrylat Monomeren mit einer quaternären

Ammoniumgruppe sein (Typ Eudragit® RS).

Bevorzugte C1- bis C4-Alkylester der Acryl- oder der Methacrylsäure sind Methylacrylat, Ethylacrylat, Butylacrylat, Butylmethacrylat und Methylmethacrylat.

Als (Meth)acrylat Monomer mit quaternären Aminogruppen wird 2-Trimethylammoniumethylmethacrylat-Chlor besonders bevorzugt.

Ein beispielhaft geeignetes Copolymer enthält 65 Gew.-% Methylmethacrylat, 30 Gew.-% Ethylacrylat und 5 Gew.-% 2-Trimethylammonium-ethylmethacrylat Chlorid (Eudragit RS).

Das schwerlösliche Polymer kann ein Polymerisat aus 93 bis 88 Gew.-% C1- bis C4-Alkylestern der Acryl- oder der Methacrylsäure und 7 bis 12 Gew.-% (Meth)acrylat Monomeren mit einer quaternären Ammoniumgruppe sein (Typ Eudragit RL).

Ein konkret geeignetes Copolymer enthält z. B. 60 Gew.-% Methylmethacrylat, 30 Gew.-% Ethylacrylat und 10 Gew.-%

2-Trimethylammoniumethlymethacrylat-Chlorid (Eudragit® RL).

Das wasserunlösliche Polymer kann eine Mischung der Polymere vom Typ

Eudragit RS und vom Typ Eudragit RL im Verhältnis 20 zu 1 bis 1 zu 20 sein.

Insbesondere geeignet sind auch Mischungen aus Eudragit RS und

Eudragit RL z. B. im Verhältnis von 20 : 1 bis 1 : 20 Gewichtsteilen.

Die pharmazeutische Zusammensetzung kann als schwerlösliches Polymer auch ein Polyvinylacetat enthalten. Als Polyvinylacetate eignen sich beispielsweise die Homopolymerisate des Vinylacetats. Weiterhin eignen sich schwerlösliche Polyvinylacetat-Copolymere, beispielsweise wasserunlösliche Copolymere aus Vinylacetat und N-Vinylpyrrolidon. Kommerziell erhältliche geeignete Polyvinylacetate sind beispielsweise Kollicoat® SR 30D oder Kollidon® SR.

Als schwerlösliche Polymere eignen sich auch Alkylcellulosen wie beispielsweise Ethylcellulose.

Anionische schwerlösliche Polymere

Weiterhin können auch anionische schwerlösliche Polymere verwendet werden. Unter anionischen Polymeren werden bevorzugt Polymere mit mindestens 5 %,

besonders bevorzugt 5 bis 75 % Monomerresten mit anionischen Gruppen verstanden. Bevorzugt sind anionische (Meth)acrylat-Copolymere.

Geeignete kommerziell erhältiche (Meth)acrylatcopolymere mit anionischen Gruppen sind beispielsweise die Eudragit®-Typen L, L100-55, S und FS.

Geeignete anionische (Meth)acrylatcopolymere sind z. B. Polymerisate aus 25 bis 95, Gew.-% C1- bis C4-Alkylestern der Acryl- oder der Methacrylsäure und 5 bis 75 Gew.-% (Meth)acrylat-Monomeren mit einer anionischen Gruppe. Entsprechende Polymere sind je nach Gehalt an anionischen Gruppen und dem Charakter der weiteren Monomere bei pH-Werten oberhalb von pH 5,O wasserlöslich und somit auch darmsaftlöslich. In der Regel addieren sich die genannten Anteile zu 100 Gew.-%.

Ein (Meth)acrylat-Monomer mit einer anionischen Gruppe kann z. B. Acrylsäure, bevorzugt jedoch Methacrylsäure sein.

Weiterhin geeignet sind anionische (Meth)acrylat Copolymere aus 40 bis 60, Gew.-%

Methacrylsäure und 60 bis 40 Gew.-% Methylmethacrylat oder 60 bis 40 Gew.-% Ethylacrylat. (Typen Eudragit L oder Eudragit L1 00-55).

Eudragit L ist ein Copolymer aus 50 Gew.-% Methylmethacrylat und 50 Gew.-% Methacrylsäure.

Eudragit L1 00-55 ist ein Copolymer aus 50 Gew.-% Ethylacrylat und 50 Gew.-% Methacrylsäure. Eudragit L 30D-55 ist eine Dispersion enthaltend 30 Gew.-% Eudragit L 100-55.

Ebenso geeignet sind anionische (Meth)acrylat Copolymere aus 20 bis 40 Gew.-% Methacrylsäure und 80 bis 60 Gew.-% Methylmethacrylat (Typ Eudragit® S). Geeignet sind weiterhin z. B. anionische (Meth)acrylat Copolymere, bestehend aus 10 bis 30 Gew.-%, Methylmethacrylat, 50 bis 70 Gew.-% Methylacrylat und 5 bis 15 Gew.-% Methacrylsäure (Typ Eudragit® FS).

Eudragit FS ist ein Copolymer aus 25 Gew.-%, Methylmethacrylat, 65 Gew.-% Methylacrylat und 10 Gew.-% Methacrylsäure. Eudragit FS 30 D ist eine Dispersion enthaltend 30 Gew.-% Eudragit® FS.

Die Copolymere bestehen bevorzugt im Wesentlichen bis ausschließlich aus den Monomeren Methacrylsäure, Methylacrylat und Ethylacrylat in den oben angegebenen Mengenanteilen.

Es können jedoch zusätzlich, ohne dass dies zu einer Beeinträchtigung der wesentlichen Eigenschaften führt, geringe Mengen im Bereich von 0 bis 10, z. B. 1 bis 5 Gew.-% weiterer vinylisch copolymerisierbarer Monomere, wie z. B. Methylmethacrylat, Butylmethacrylat, Butylacrylat oder Hydroxyethylmethacrylat enthalten sein.

Die Copolymerisate können nach gängigen Verfahren der radikalischen Polymerisation kontinuierlich oder diskontinuierlich (Batch-Verfahren) in Gegenwart radikalbildender Initiatoren und gegebenenfalls Reglern zur Einstellung des Molekulargewicht in Substanz, in Lösung, durch Perlpolymerisation oder in Emulsion hergestellt werden. Das mittlere Molekulargewicht Mw (Gewichtsmittel, bestimmt z. B. durch Messung der Lösungsviskosität) kann z. B. im Bereich von 80.000 bis 1.000.000 (glmol) liegen. Bevorzugt ist die Emulsionspolymerisation in wässriger Phase in Gegenwart wassergelöster Initiatoren und (vorzugsweise anionischer) Emulgatoren. Im Falle der Substanzpolymerisation kann das Copolymer in fester Form durch Brechen, Extrusion, Granulieren oder Heißabschlag erfolgen.

Weiterhin eignet sich als anionisches Polymer Hydroxypropylmethylcelluloseacetatsuccinat, ein magensaftresistentes Polymer, das im basischen löslich ist.

### Basische schwerlösliche Polymere

Es können auch basische Polymere wie basische Meth(acrylate) oder Chitosan eingesetzt werden.. Ein Beispielfür ein entsprechendes kommerziell erhältliches Polymer ist Eudragit® E oder EPO, das ein Copolymer aus Methylmethacrylat, Butylmethacrylat und Dimethylaminoethylmethacrylat darstellt.

Die Herstellung der festen Zubereitungen kann mit an sich bekannten Methoden erfolgen.

Gemäß einer Ausführungsform werden alle Inhaltsstoffe der Zubereitungen zunächst miteinander in einem geeigneten Lösungsmittel in Lösung gebracht und das Lösungsmittel anschliessend entfernt. Als Lösungsmittel können alle in der pharmazeutischen Technologie üblichen Lösungmittel verwendet werden, beispielswiese Ethanol, Isoporanol, n-Butanol, Isobutanol, Essigsäureethylester, Aceton oder Dimethylformamid. Die Entfernung der Lösungsmittel kann über alle möglichen Arten von Trocknungsprozessen erfolgen z.B. über Sprühtrocknung, Wirbelschichttrocknung, Walzentrocknung, Trocknung unter Anwendung überkritischer Gase, Gefriertrocknung, Abdampfen.

Gemäß einer bevorzugten Verfahrensweise werden die festen Zubereitungen durch Extrusion hergestellt.

Die Polymere können dem Extruder sowohl in pulverförmiger Form als auch in Form von Lösungen oder Dispersionen zugeführt werden.

Die Dispersionen oder Lösungen der Polymeren können durch Entfernung des Dispersions- oder Lösemittels im Extruder im schmelzeflüssigen Zustand und Abkühlen der Schmelze in eine feste Form überführt werden.

Die so erhaltene Schmelze kann dann abgekühlt und granuliert werden. Hierzu erfolgt ein sogenannter Heißabschlag oder Abkühlung unter Luft oder Schutzgas beispielsweise auf ein Teflon- oder Kettenband und anschließende Granulierung des erkalteten Schmelzestranges. Es kann aber auch eine Abkühlung in einem Lösungsmittel erfolgen, in dem die Polymere nicht signifikant löslich sind.

Die folgenden Methoden A-E können grundsätzlich verwendet werden:

| | |
|---|---|
| A | physikalische Pulvermischung aus Polymeren und Wirkstoff und Zuführung dieser Pulvermischung in den Extruder |
| B | Zuführung des Wirkstoffes über einen separaten Bypass in ungeschmolzene Polymermischung |
| C | Zuführung des Wirkstoffes über eine Seitendosierung in geschmolzene Polymere |
| D | Polymerlösung mit darin dispergiertem oder gelöstem Wirkstoff in teilentgaste Polymerschmelze oder ungeschmolzene Polymermischung; |
| E | Verfahren nach A-D, wobei zusätzlich Lösungsmittel in den Extruder eingebracht und wieder verdampft wird |

Für das erfindungsgemäße Verfahren eignen sich prinzipiell die üblichen, dem Fachmann bekannten Extrudertypen. Diese umfassen üblicherweise ein Gehäuse, eine Antriebseinheit mit Getriebe sowie eine Verfahrenseinheit, die aus der oder den mit den Schneckenelementen bestückten Extruderwellen besteht, wobei in diesem Falle modularer Aufbau vorausgesetzt wird.

Der Extruder besteht aus mehreren Abschnitten, die jeweils bestimmten Verfahrenseinheiten zuzuordnen sind. Jeder dieser Abschnitte besteht aus einem oder mehreren Zylindern (Zylinderblöcken) als kleinste unabhängige Einheit -und den zugehörigen Schneckenabschnitten mit den der Verfahrensaufgabe entsprechenden Schneckenelementen.

Die einzelnen Zylinder sollen beheizbar sein. Weiterhin können die Zylinder auch für eine Kühlung ausgelegt sein, beispielsweise für Kühlung mit Wasser. Bevorzugt sind die einzelnen Zylinderblöcke voneinander unabhängig heiz- und kühlbar, so dass auch längs der Extrusionsrichtung unterschiedliche Temperaturzonen eingestellt werden können

Der Extruder ist vorteilhafterweise als gleichsinnig drehender Zweischneckenextruder ausgeführt. Die Schneckenkonfiguration kann dabei je nach Produkt unterschiedlich stark scherend sein. Knetelemente müssen insbesondere in der Aufschmelzzone eingesetzt werden. Dabei können auch rückläufige Knetelemente eingesetzt werden.

Geeignete Zweischneckenextruder können einen Schneckendurchmesser von 16 bis70 mm und eine Länge von25 bis 40 D aufweisen..

Der gesamte Extruder ist aus Zylinderblöcken, die einzeln temperierbar sind, aufgebaut. Die ersten zwei Zylinder können zwecks besseren Materialeinzugs temperiert sein. Ab dem dritten Zylinder wird vorzugsweise eine konstante Temperatur eingestellt, die materialspezifisch zu wählen ist und insbesondere vom Schmelzpunkt des eingesetzten Wirkstoffes und der Glasübergangstemperatur des Polymers abhängt. Die resultierende Produkttemperatur ist üblicherweise jedoch vom Schergrad des eingesetzten Schneckenelements abhängig und kann mitunter 20-30°C höher sein als die eingestellte Zylindertemperatur.

Nach der Aufschmelzzone kann eine Entgasungszone nachgeschaltet werden, die vorteilhafterweise mit Umgebungsdruck betrieben wird.

Die eingesetzten Runddüsen können einen Durchmesser von 0.5 bis 5 mm aufweisen. Andere Düsenformen, wie Schlitzdüsen können ebenfalls eingesetzt werden, vor allem dann wenn ein größerer Materialdurchsatz angestrebt wird.

Die zwei gleichläufig drehenden Schnecken sind so ausgelegt, dass nach einer Einzugszone bestehend aus Förderelementen in der dritten Heizzone schon Knetblöcke mit einer anschließenden Stauscheibe eingesetzt werden. Nach einer kurzen Entspannungszone aus Förderelementen, wird das nun geschmolzene Material erneut in einer Knetzone durchmischt. Darauf folgt eine Förderelementzone mit anschließenden Knetelementen. Es folgt eine Förderelementzone mit anschließender Knetzone. Schließlich wird der Austrag des Materials mit einer Förderelementzone sichergestellt.

Die resultierenden Extrudatstränge können mit einem Granulator zu Granulatkörnern verarbeitet werden und diese können wiederum weiter zu einem Pulver zerkleinert (gemahlen) werden. Das Granulat oder Pulver kann in Kapseln gefüllt oder unter Anwendung üblicher Tablettierhilfsstoffe zu Tabletten verpresst werden. Hierbei können auch weitere die Freisetzung steuernde Hilfsstoffe eingesetzt werden.

Ferner ist es möglich, während der Extrusion Wasser, organische Lösungsmittel, Puffersubstanzen oder Weichmacher einzusetzen. Insbesondere Wasser oder flüchtige

Alkohole bieten sich hierfür an. Dieses Verfahren ermöglicht die Umsetzung bei niedrigerer Temperatur. Die Lösungsmittel- bzw. Weichmachermengen liegen üblicherweise zwischen 0 und 30% der extrudierbaren Masse. Das Wasser oder Lösungsmittel kann schon durch eine Entgasungsstelle im Extruder bei Normaldruck oder durch Anlegen von Vakuum entfernt werden. Alternativ verdampfen diese Komponenten, wenn das Extrudat den Extruder verlässt und der Druck sich auf Normaldruck reduziert. Im Falle von schwerer flüchtigen Komponenten kann das Extrudat entsprechend nachgetrocknet werden.

In einer besonderen Variante des Herstellungsverfahrens wird direkt im Anschluß an die Extrusion die thermoplastische Masse zu einem tablettenähnlichen Komprimat kalandriert, das die endgültige Darreichungsform darstellt. In dieser Variante kann es sinnvoll sein, weitere Bestandteile wir z.B. Polymere zur Einstellung der Glasübergangstemperatur und der Schmelzviskosität, Sprengmittel, Solubilisatoren, Weichmacher, Farbstoffe, Geschmacksstoffe, Süßstoffe etc. schon vor oder während der Extrusion zuzusetzen. Prinzipiell können diese Stoffe auch verwendet werden, wenn das Extrudat zunächst zerkleinert und danach zu Tabletten verpresst wird.

Zur Einstellung der Glasübergangstemperatur der Formulierung können wasserlösliche Polymere mit hoher Glasübergangstemperatur wie z.B. Polyvinylpyrrolidon mit K-Werten von 17 - 120, Hydroxyalkylcellulosen oder Hydroxyalkylstärken eingesetzt werden. Eine zu hohe Glasübergangstemperatur der Formulierung kann durch Zusatz von Weichmachern erniedrigt werden. Hierfür sind prinzipiell alle Weichmacher geeignet, die auch für pharmazeutische Coatings eingesetzt werden, wie z.B. Triethylcitrat, Tributylcitrat, Acetyltributylcitrat, Triacetin, Propylenglycol, Polyethylenglycol 400, Dibutylsebacat, Glycerolmonostearat, Laurinsäure, Cetylstearylalkohol.

Weiterhin können auch zusätzlich Tenside, die die Schmelzviskosität und damit die Extrusionstemperatur herabsetzen, in die Zubereitungen eingearbeitet werden. Diese Stoffe können auch die mögliche Kristallisation positiv beeinflussen sowie eine bessere Benetzung der Formulierung und eine schnellere Auflösung herbeiführen. Geeignete Stoffe sind ionische und nichtionische Tenside wie beispielsweise Solutol® HS 15 (Macrogol-15-Hydroxystearat), Tween® 80, polyoxyethylierte Fettsäurederivate wie Cremophor®RH40 (polyoxyl 40 Hydrogenated Castor Oil, USP), Cremophor EL (Polyoxyl 35 Castor Oil, USP), Poloxamere, Docusat Natrium oder Natriumlaurylsulfat.

Die noch plastische Mischung wird vorzugsweise durch eine Düse extrudiert, abgekühlt und zerkleinert. Zur Zerkleinerung eignen sich grundsätzlich alle üblichen hierfür bekannten Techniken wie Heiss- oder Kaltabschlag.

Das Extrudat wird beispielsweise mit rotierenden Messern oder mit einem Luftstrahl abgeschlagen und anschließend mit Luft oder unter Schutzgas abgekühlt.

Es ist auch möglich, das Extrudat als Schmelzestrang auf einem gekühlten Band (Edelstahl, Teflon, Kettenband) abzulegen und nach Erstarrung zu granulieren.

Anschließend kann das Extrudat gegebenenfalls gemahlen werden. Die Zubereitungen werden als riesel- und fließfähige wasserlösliche Pulver gewonnen. Bevorzugt werden Partikelgrößen von 20 bis 250 µm eingestellt.

Weiterhin ist es auch möglich die plastische Mischung aus Polymer und aktiver Substanz durch Spritzguss zu verarbeiten.

Überraschenderweise weisen die erfindungsgemäßen Formulierungen eine erheblich verbesserte Stabilität auf, das heißt der Wirkstoff bleibt in molekulardispersem oder kolloiddispersem amorphem Zustand in der Formulierung und kristallisiert nicht aus. Dadurch verändern sich auch die Freisetzungseigenschaften nicht über die Zeit. Sofern die Teilchengröße der Extrudate oberhalb von 300µm liegt, zeigt sich die Wirkung des in Wasser schwerlöslichen Polymers auf die Freisetzung. Das bedeutet in diesen Fällen ist die Freisetzung entsprechend dem Zusatz retardiert oder magensaftresistent. Wird das Extrudat aber zerkleinert, so dass Teilchengrößen unter 100µm liegen, wird überraschenderweise eine schnelle Freisetzung erzielt selbst bei Verwendung von magensaftresistenten oder retardierenden Polymeren.

Die erfindungsgemäßen Zubereitungen weisen eine höhere Bioverfügbarkeit des Wirkstoffes auf.

Die nach dem erfindungsgemäßen Verfahren erhaltenen Zubereitungen lassen sich grundsätzlich auf allen Gebieten einsetzen, bei denen in Wasser nur schwerlösliche oder unlösliche Substanzen entweder in wässrigen Zubereitungen zum Einsatz kommen sollen oder ihre Wirkung in wässrigem Milieu entfalten sollen.

Im Sinne der vorliegenden Erfindung sind unter in Wasser schwerlöslichen Substanzen vorzugsweise biologisch aktive Substanzen wie pharmazeutische Wirkstoffe für Mensch und Tier, kosmetische oder agrochemische Wirkstoffe oder Nahrungsergänzungsmittel oder diätetische Wirkstoffe zu verstehen.

Weiterhin kommen als zu solubilisierende schwerlösliche Substanzen auch Farbstoffe wie anorganische oder organische Pigmente in Betracht.

Die Extrusionstemperaturen liegen üblicherweise im Bereich von 50 bis 260°C, vorzugsweise im Bereich von 70 - 200°C. Die Temperaturuntergrenze richtet sich nach der Zusammensetzung der zu extrudierenden Mischungen und den jeweils zu verabeitenden schwerlöslichen Substanzen. Zumindest ein Teil der Mischung muß bei der gewählten Temperatur plastifizierbar sein. Die Temperaturobergrenze ist definiert durch die Zersetzung des Wirkstoffes oder der Polymeren.

Die verwendeten pharmazeutischen Wirkstoffe sind in Wasser unlösliche bzw. wenig lösliche Substanzen gemäß der Definition nach DAB 9.

Gemäß DAB 9 (Deutsches Arzneimittelbuch) erfolgt die Einstufung der Löslichkeit von Stoffen wie folgt: wenig löslich (löslich in 30 bis 100 Teilen Lösungsmittel); schwer löslich (löslich in 100 bis 1000 Teilen Lösungsmittel); praktisch unlöslich (löslich in mehr als 10000 Teilen Lösungsmittel), jeweils bezogen auf ein Teil zu lösendem Stoff bei 20 °C.

Die Wirkstoffe können aus jedem Indikationsbereich kommen.

Als Beispiele seien hier Benzodiazepine, Antihypertensiva, Vitamine, Cytostatika - insbesondere Taxol, Anästhetika, Neuroleptika, Antidepressiva, antiviral wirksame Mittel wie beispielsweise Anti-HIV wirksame Mittel, Antibiotika, Antimykotika, Antidementiva, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regutatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, , Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel genannt.

Besonders bevorzugt sind von den oben genannten pharmazeutischen Zubereitungen solche, bei denen es sich um oral applizierbare Formulierungen handelt.

Der Gehalt an der Mischung aus Polyether-Copolymer und schwerlöslichem Polymer in der pharmazeutischen Zubereitung liegt, abhängig vom Wirkstoff, im Bereich von 1 bis 99 Gew.-%, bevorzugt 5 bis 80 Gew.-%, besonders bevorzugt 10 bis 70 Gew.-%.

Zur Herstellung von pharmazeutischen Darreichungsformen wie beispielsweise Tabletten können die Extrudate mit üblichen pharmazeutischen Hilfsstoffen versetzt werden.

Dabei handelt es sich um Stoffe aus der Klasse der Füllstoffe, Weichmacher, Löslichkeitsvermittler, Bindemittel, Silikate sowie Spreng- und Adsorptionsmittel, Schmiermittel, Fließmittel, Farbstoffe, Stabilisatoren wie Antioxidantien, Netzmittel, Konservierungsmittel, Formentrennmittel, Aromen oder Süßstoffe, bevorzugt um Füllstoffe, Weichmacher und Löslichkeitsvermittler.

Als Füllstoffe können z.B. anorganische Füllstoffe wie Oxide von Magnesium, Aluminium, Silicium, Titan- oder Calciumcarbonat, Calcium- oder Magnesiumphosphate oder organische Füllstoffe wie Lactose, Saccharose, Sorbit, Mannit zugesetzt werden.

Als Weichmacher eignen sich beispielsweise Triacetin, Triethylcitrat, Glycerolmonostearat, niedermolekulare Polyethylenglykole oder Poloxamere.

Als zusätzliche Löslichkeitvermittler eignen sich grenzflächenaktive Substanzen mit einem HLB-Wert (HydrophilicLipophilicBalance) größer 11, beispielsweise mit 40 Ethylenoxid-Einheitten ethoxiliertes hydriertes Ricinusöl (Cremophor® RH 40), mit 35 Ethylenoxid-Einheiten ethoxiliertes Ricinusöl (Cremophor eL), Polysorbat 80, Poloxamere, Docusat-Natrium oder Natriumlaurylsulfat.

Als Schmiermittel können Stearate von Aluminium, Calcium, Magnesium und Zinn, sowie Magnesiumsilikat, Silikone und ähnliche verwendet werden.

Als Fließmittel können beispielsweise Talk oder kolloidales Siliciumdioxid eingesetzt werden.

Als Bindemittel eignet sich zum Beispiel mikrokristalline Cellulose.

Zur zusätzlichen Einstellung einer verzögerten Freisetzung können den Tablettiermischungen auch noch weitere in Wasser schwerlösliche Polymere zugegeben werden.

Als Sprengmittel können quervernetztes Polyvinylpyrrolidon oder quervernetzte Natriumcarboxymethylstärke sein. Stabilisatoren können sein Ascorbinsäure oder Tocopherol.

Farbstoffe sind z.B. Eisenoxide, Titandioxid, Triphenylmethanfarbstoffe, Azofarbstoffe, Chinolinfarbstoffe, Indigotinfarbstoffe, Carotinoide, um die Darreichungsformen einzufärben, Opakisierungsmittel wie Titandioxid oder Talkum, um die Lichtdurchlässigkeit zu erhöhen und um Farbstoffe einzusparen.

Neben der Anwendung in der Kosmetik und Pharmazie eignen sich die erfindungsgemäß hergestellten Zubereitungen auch für den Einsatz im Lebensmittelbereich, zum Beispiel für die Einarbeitung von schwer wasserlöslichen oder wasserunlösliche Nähr-, Hilfs- oder Zusatzstoffen, wie z.B. fettlösliche Vitamine oder Carotinoide. Als Beispiele seien mit Carotinoiden gefärbte Getränke genannt.

Die Anwendung der erfindungsgemäß erhaltenen Zubereitungen in der Agrochemie kann u.a. Formulierungen umfassen, die Pestizide, Herbizide, Fungizide oder Insektizide enthalten, vor allem auch solche Zubereitungen von Pflanzenschutzmitteln, die als Spritz- oder Gießbrühen zum Einsatz kommen.

Mit Hilfe des erfindungsgemäßen Verfahrens können sogenannte feste Lösungen mit schwerlöslichen Substanzen erhalten werden. Als feste Lösungen werden erfindungsgemäß Systeme bezeichnet, in denen keine kristallinen Anteile der schwerlöslichen Substanz zu beobachten sind.

Bei visueller Begutachtung der stabilen festen Lösungen sind keine amorphen Bestandteile zu erkennen. Die visuelle Begutachtung kann mit einem Lichtmikroskop sowohl mit als auch ohne Polarisationsfilter bei 40facher Vergrößerung erfolgen.

Weiterhin können die Zubereitungen auch mit Hilfe XRD (X-Ray Diffraction; Röntgendiffraktometrie) und

DSC (Differential Scanning Calorimetry) auf Kristallinität oder Amorphizität untersucht werden. Die nach dem erfindungsgemäßen Verfahren erhaltenen Zubereitungen liegen wie gesagt amorph vor, was bedeutet, dass die kristallinen Anteile der biologisch aktiven Substanz kleiner 5 Gew.-% betragen. Vorzugsweise wird der amorphe Zustand mittels DSC oder XRD überprüft. Solch ein amorpher Zustand kann auch als röntgenamorpher Zustand bezeichnet werden.

Das erfindungsgemäße Verfahren erlaubt die Herstellung von stabilen Zubereitungen mit hoher Wirkstoffbeladung und guter Stabilität bezüglich des amorphen Zustand der schwerlöslichen Substanz.

Das erfindungsgemäße Verfahren erlaubt die Herstellung von stabilen Zubereitungen mit hoher Wirkstoffbeladung.

### Beispiele

### Herstellung von Polymer 1

In einer Rührapparatur wurde die Vorlage ohne die Teilmenge von Zulauf 2 unter einer N₂-Atmosphäre auf 77°C aufgeheizt. Wenn die Innentemperatur von 77°C erreicht war, wurde die Teilmenge von Zulauf 2 zugegeben und 15 min anpolymerisiert. Anschließend wurden Zulauf 1 in 5h und Zulauf 2 in 2 h zudosiert. Nachdem alle Zuläufe zudosiert waren, wurde das Reaktionsgemisch noch 3h nachpolymerisiert. Nach der Nachpolymerisation wurde die Lösung auf einen Feststoffgehalt von 50 Gew.-% eingestellt.
Vorlage: 25 g Ethylacetat
   104,0 g PEG 6000,
   1,0 g von Zulauf 2
Zulauf 1: 240 g Vinylacetat
Zulauf 2: 456 g Vinylcaprolactam
   240 g Ethylacetat
Zulauf 3: 10,44 g tert-Butylperpivalat (75 gew.%-ig in Aliphatengemisch)
   67,90 g Ethylacetat

Anschliessend wurde das Lösungsmittel durch ein Srpühverfahren entfernt und ein pulverförmiges Produkt erhalten. Der K-Wert betrug 16 , gemessen 1 gew.-%ig in Ethanol

Der Zweischneckenextruder, der für die Herstellung der in den nachfolgenden Beispielen beschriebenen Formulierungen eingesetzt wurde, wies einen Schneckendurchmesser von 16 mm und eine Länge von 40D auf. Der gesamte Extruder war aus 8 einzeln temperierbaren Zylinderblöcken aufgebaut. Die ersten zwei Zylinder wurden zwecks besseren Materialeinzugs bei 20°C bzw. bei 70°C temperiert. Ab dem dritten Zylinder wurde eine konstante Temperatur eingestellt,

Die hergestellten festen Lösungen wurden mittels XRD (Röntgendiffraktometrie) und DSC (Differential Scanning Calorimetry) auf Kristallinität bzw. Amorphizität unter Verwendung folgender Geräte und Bedingungenuntersucht:

### XRD

Messgerät: Diffraktometer D 8 Advance mit 9-fach Probenwechsler (Fa.Bruker/AXS) Messart: θ- θ Geometrie in Reflexion
Winkelbereich 2 Theta: 2-80°
Schrittweite: 0,02°
Messzeit pro Winkelschritt: 4,8s
Divergence Slit: Göbelspiegel mit 0,4 mm Steckblende
Antiscattering Slit: Sollerspalt
Detektor: Sol-X Detektor
Temperatur: Raumtemperatur
Generatoreinstellung: 40kV/50mA

### DSC

DSC Q 2000 der Fa. TA -Instruments
Parameter:
Einwaage ca. 8,5 mg
Heizrate: 20K/min

Die Wirkstofffreisetzung erfolgte nach USP Apparatur (Paddle Methode) 2, 37°C, 50UpM (BTWS 600, Pharmatest). Die Extrudatstränge wurden mittels eines Granulators auf eine Länge von 3mm zerteilt und in Hartgelatinekapseln gefüllt. Die Detektion des freigesetzen Wirkstoffes erfolgte per UV Spektroskopie (Lamda-2, Perkin Elmer)

### Beispiel 1

1200g Polymer 1, 400g Eudragit E PO und 400g Celecoxib (Schmelzpunkt 162°C) wurden in einen Turbulamischbehälter eingewogen und 10 Minuten im Turbulamischer T10B gemischt.

Die Mischung wurde unter folgenden Bedingungen extrudiert:
- Zonentemperatur 1. Zylinder: 20°C; 2. Zylinder: 40°C
- Zonentemperatur ab dem 3. Zylinder: 140°C
- Schneckendrehzahl 200 UpM
- Durchsatz:500g/h
- Düsendurchmesser 3mm

Die festen Lösungen wurden mit XRD und per DSC untersucht und als amorph befunden. Nach 1 h in 0,1 normaler HCl waren 95% Wirkstoff freigesetzt.

Nach 6 Monaten Lagerung bei 30°C/ 70 % relative Feuchte waren die Zubereitungen immer noch amorph.

### Beispiel 2

800g Polymer 1, 800g Eudragit E PO und 400g Naproxen (Schmelzpunkt 157°C) wurden in einen Turbulamischbehälter eingewogen und 10 Minuten im Turbulamischer T10B gemischt.

Die Mischung wurde unter folgenden Bedingungen extrudiert:
- Zonentemperatur 1. Zylinder: 20°C; 2. Zylinder: 40°C
- Zonentemperatur ab dem 3. Zylinder: 120°C
- Schneckendrehzahl 200 UpM
- Durchsatz: 600g/h
- Düsendurchmesser 3mm

Die festen Lösungen wurden mit XRD und per DSC untersucht und als amorph befunden. Nach 1 h in 0,1 normaler HCl waren 89% Wirkstoff freigesetzt.

### Beispiel 3

1200g Polymer 1, 400g Eudragit E PO, 40g Natriumlaurylsulfat und 400g Itraconazol (Schmelzpunkt 166°C) wurden in einen Turbulamischbehälter eingewogen und 10 Minuten im Turbulamischer T10B gemischt.

Die Mischung wurde unter folgenden Bedingungen extrudiert:
- Zonentemperatur 1. Zylinder: 20°C; 2. Zylinder: 40°C
- Zonentemperatur ab dem 3. Zylinder: 140°C
- Schneckendrehzahl 200 UpM
- Durchsatz:800g/h
- Düsendurchmesser 3mm

Die festen Lösungen wurden mit XRD und per DSC untersucht und als amorph befunden. Nach 1 h in 0,1 normaler HCl waren 99% Wirkstoff freigesetzt.

Nach 6 Monaten Lagerung bei 30°C/ 70 % rel. Feuchte waren die Zubereitungen immer noch amorph.

### Beispiel 4

600g Polymer 1, 1000g Kollidon SR und 400g Fenofibrat (Schmelzpunkt 81 °C) wurden in einen Turbulamischbehälter eingewogen und 10 Minuten im Turbulamischer T10B gemischt.

Die Mischung wurde unter folgenden Bedingungen extrudiert:
- Zonentemperatur 1. Zylinder: 20°C; 2. Zylinder: 40°C
- Zonentemperatur ab dem 3. Zylinder: 110°C
- Schneckendrehzahl 200 UpM
- Durchsatz:1000g/h
- Düsendurchmesser 3mm

Die festen Lösungen wurden mit XRD und per DSC untersucht und als amorph befunden. Die Freisetzung des Wirkstoffs in 0,1 normaler HCl nach 2 h betrug unter 20%. Nach Umpufferung auf pH 6,8 wurde dies für weitere 10 h wonach insgesamt 80% Wirkstoff freigesetzt wurde.

Nach 6 Monaten Lagerung bei 30°C/ 70 5 rel. Feuchte waren die Zubereitungen immer noch amorph.

### Beispiel 5

600g Polymer 1, 1000g Eudragit S 100 und 400g Cinnarizin (Schmelzpunkt 122°C) wurden in einen Turbulamischbehälter eingewogen und 10 Minuten im Turbulamischer T10B gemischt. Über eine Hubkolbenpumpe wurde Triethylcitrat in den Extruder eingespeist.

Die Mischung wurde unter folgenden Bedingungen extrudiert:
- Zonentemperatur 1. Zylinder: 20°C; 2. Zylinder: 40°C
- Zonentemperatur ab dem 3. Zylinder: 130°C
- Schneckendrehzahl 100 UpM
- Durchsatz:800g/h
- Flüssigkeitsdosierung: 80g/h
- Düsendurchmesser 3mm

Die festen Lösungen wurden mit XRD und per DSC untersucht und als amorph befunden. Die Freisetzung des Wirkstoffs in 0,1 normaler HCl nach 2h betrug unter 10%, nach Umpufferung auf pH 6,8 wurden 100% freigesetzt.

Nach 6 Monaten Lagerung bei 30°C / 70 % rel. Feuchte waren die Zubereitungen immer noch amorph.

### Beispiel 6

400g Polymer 1, 1200g Eudragit E PO und 400g Carbamazepin (Schmelzpunkt 192°C) wurden in einen Turbulamischbehälter eingewogen und 10 Minuten im Turbulamischer T10B gemischt.

Die Mischung wurde unter folgenden Bedingungen extrudiert:
- Zonentemperatur 1. Zylinder: 20°C; 2. Zylinder: 40°C
- Zonentemperatur ab dem 3. Zylinder: 160°C
- Schneckendrehzahl 200 UpM
- Durchsatz: 600g/h
- Düsendurchmesser 3mm

Die festen Lösungen wurden mit XRD und per DSC untersucht und als amorph befunden. Nach 1 h in 0,1 normaler HCl waren 95% Wirkstoff freigesetzt.

Nach 6 Monaten Lagerung bei 30°C/ 70 % rel. Feuchte waren die Zubereitungen immer noch amorph.

### Beispiel 7

600g Polymer 1, 1000g Eudragit L 100-55 und 400g Loperamid (Schmelzpunkt 223°C) wurden in einen Turbulamischbehälter eingewogen und 10 Minuten im Turbulamischer T10B gemischt.

Die Mischung wurde unter folgenden Bedingungen extrudiert:
- Zonentemperatur 1. Zylinder: 20°C; 2. Zylinder: 40°C
- Zonentemperatur ab dem 3. Zylinder: 170°C
- Schneckendrehzahl 200 UpM
- Durchsatz:1000g/h
- Düsendurchmesser 3mm

Die festen Lösungen wurden mit XRD und per DSC untersucht und als amorph befunden. Die Freisetzung des Wirkstoffs in 0,1 normaler HCl nach 2h betrug unter 10%, nach Umpufferung auf pH 6,8 wurden 98% freigesetzt.

Nach 6 Monaten Lagerung bei 30°C/ 70 % rel. Feuchte waren die Zubereitungen immer noch amorph.

### Beispiel 8

600g Polymer 1, 500g Eudragit RS PO, 500g Eudragit RL PO und 400g Clotrimazol (Schmelzpunkt 148°C) wurden in einen Turbulamischbehälter eingewogen und 10 Minuten im Turbulamischer T10B gemischt.

Die Mischung wurde unter folgenden Bedingungen extrudiert:
- Zonentemperatur 1. Zylinder: 20°C; 2. Zylinder: 40°C
- Zonentemperatur ab dem 3. Zylinder: 150°C
- Schneckendrehzahl 100 UpM
- Durchsatz:700g/h
- Düsendurchmesser 3mm

Die festen Lösungen wurden mit XRD und per DSC untersucht und als amorph befunden. Die Freisetzung des Wirkstoffs in Phosphatpuffer pH 6,8 betrug 20% nach 2h, nach 10h waren 84% des ursprünglich eingesetzten Wirkstoffs freigesetzt.

Nach 6 Monaten Lagerung bei 30°C waren die Zubereitungen immer noch amorph.

### Beispiel 9

600g Polymer 1, 1000g HPMCAS (Hydroxypropyl methylcellulose acetat succinat) und 400g Cinnarizin (Schmelzpunkt 122°C) wurden in einen Turbulamischbehälter eingewogen und 10 Minuten im Turbulamischer T10B gemischt.

Die Mischung wurde unter folgenden Bedingungen extrudiert:
- Zonentemperatur 1. Zylinder: 20°C; 2. Zylinder: 40°C
- Zonentemperatur ab dem 3. Zylinder: 140°C
- Schneckendrehzahl 100 UpM
- Durchsatz:800g/h
- Düsendurchmesser 3mm

Die festen Lösungen wurden mit XRD und per DSC untersucht und als amorph befunden. Die Freisetzung des Wirkstoffs in 0,1 normaler HCl nach 2h betrug unter 10%, nach Umpufferung auf pH 6,8 wurden 90% freigesetzt.

Nach 6 Monaten Lagerung bei 30°C/ rel. Feuchte war die Zubereitungen immer noch amorph.

### Beispiel 10

600g Polymer 1, 500g Eudragit RS PO, 500g Eudragit RL PO und 400g Piroxicam (Schmelzpunkt 199°C) wurden in einen Turbulamischbehälter eingewogen und 10 Minuten im Turbulamischer T10B gemischt.

Die Mischung wurde unter folgenden Bedingungen extrudiert:
- Zonentemperatur 1. Zylinder: 20°C; 2. Zylinder: 40°C
- Zonentemperatur ab dem 3. Zylinder: 170°C
- Schneckendrehzahl 100 UpM
- Durchsatz:700g/h
- Düsendurchmesser 3mm

Die festen Lösungen wurden mit XRD und per DSC untersucht und als amorph befunden. Die Freisetzung des Wirkstoffs in Phosphatpuffer pH 6,8 betrug 20% nach 2h, nach 10h waren 93% des ursprünglich eingesetzten Wirkstoffs freigesetzt.

Nach 6 Monaten Lagerung bei 30°C/ 70 % rel. Feuchte waren die Zubereitungen immer noch amorph.

### Beispiel 11

600g Polymer 1, 1000g Ethylcellulose und 400g Felodipin (Schmelzpunkt 145°C) wurden in einen Turbulamischbehälter eingewogen und 10 Minuten im Turbulamischer T10B gemischt. Über eine Hubkolbenpumpe wurde Eudragit NE 40D in den Extruder eingespeist.

Die Mischung wurde unter folgenden Bedingungen extrudiert:
- Zonentemperatur 1. Zylinder: 20°C; 2. Zylinder: 40°C
- Zonentemperatur ab dem 3. Zylinder: 140°C
- Schneckendrehzahl 100 UpM
- Durchsatz: 800g/h
- Flüssigkeitsdosierung: 60g/h
- Düsendurchmesser 3mm

Die festen Lösungen wurden mit XRD und per DSC untersucht und als amorph befunden. Die Freisetzung des Wirkstoffs in Phosphatpuffer pH 6,8 betrug 31 % nach 2h, nach 10h waren 79% des ursprünglich eingesetzten Wirkstoffs freigesetzt.

Nach 6 Monaten Lagerung bei 30°C/ 70 % rel. Feuchte waren die Zubereitungen immer noch amorph.

### Beispiel 12

600g Polymer 1, 1000g Eudragit RS PO und 400g Itraconazol (Schmelzpunkt 166°C) wurden in einen Turbulamischbehälter eingewogen und 10 Minuten im Turbulamischer T10B gemischt.

Die Mischung wurde unter folgenden Bedingungen extrudiert:
- Zonentemperatur 1. Zylinder: 20°C; 2. Zylinder: 40°C
- Zonentemperatur ab dem 3. Zylinder: 150°C
- Schneckendrehzahl 100 UpM
- Durchsatz:800g/h
- Düsendurchmesser 3mm

Die festen Lösungen wurden mit XRD und per DSC untersucht und als amorph befunden. Die Freisetzung des Wirkstoffs in Phosphatpuffer pH 6,8 betrug 27% nach 2h, nach 10h waren 82% des ursprünglich eingesetzten Wirkstoffs freigesetzt.

Nach 6 Monaten Lagerung bei 30°C/ 70 % rel. Feuchte waren die Zubereitungen immer noch amorph.

### Beispiel 13

400g Polymer 1, 1200g Eudragit RL PO und 400g Carbamazepin (Schmelzpunkt 192°C) wurden in einen Turbulamischbehälter eingewogen und 10 Minuten im Turbulamischer T10B gemischt.

Die Mischung wurde unter folgenden Bedingungen extrudiert:
- Zonentemperatur 1. Zylinder: 20°C; 2. Zylinder: 40°C
- Zonentemperatur ab dem 3. Zylinder: 160°C
- Schneckendrehzahl 100 UpM
- Durchsatz:800g/h
- Düsendurchmesser 3mm

Die festen Lösungen wurden mit XRD und per DSC untersucht und als amorph befunden. Die Freisetzung des Wirkstoffs in HCl nach 2h betrug unter 20%. Nach Umpufferung auf pH 6,8 wurden 75% nach 10h freigesetzt.

Nach 6 Monaten Lagerung bei 30°C/70 5 rel. Feuchte waren die Zubereitungen immer noch amorph.

### Beispiel 14

1200g Polymer 1, 400g Eudragit E PO, 20g Docusat-Natrium und 400g Fenofibrat (Schmelzpunkt 81 °C) wurden in einen Turbulamischbehälter eingewogen und 10 Minuten im Turbulamischer T10B gemischt.

Die Mischung wurde unter folgenden Bedingungen extrudiert:
- Zonentemperatur 1. Zylinder: 20°C; 2. Zylinder: 40C
- Zonentemperatur ab dem 3. Zylinder: 120°C
- Schneckendrehzahl 100 UpM
- Durchsatz:800g/h
- Düsendurchmesser 3mm

Die festen Lösungen wurden mit XRD und per DSC untersucht und als amorph befunden. Die Freisetzung des Wirkstoffs in HCl nach 2h betrug 83%.

Nach 6 Monaten Lagerung bei 30°C war die Zubereitungen immer noch amorph.

### Beispiel 15

600g Polymer 1, 500g Kollidon SR, 500g Ethylcellulose, 20g Cremophor RH40 und 400g Clotrimazol (Schmelzpunkt 148°C) wurden in einen Turbulamischbehälter eingewogen und 10 Minuten im Turbulamischer T10B gemischt.

Kollidon SR: physikalische Mischung aus 80 Gew.-% Polyvinylacetat, 19 gew.-% Polyvinylpyrrolidon k30, 0.8 Gew.-% Natriumlaurylsulfat, 0.2 Gew.-% Siliciumdioxid Die Mischung wurde unter folgenden Bedingungen extrudiert:
- Zonentemperatur 1. Zylinder: 20°C; 2. Zylinder: 40°C
- Zonentemperatur ab dem 3. Zylinder: 160°C
- Schneckendrehzahl 100 UpM
- Durchsatz:700g/h
- Düsendurchmesser 3mm

Die festen Lösungen wurden mit XRD und per DSC untersucht und als amorph befunden. Die Freisetzung des Wirkstoffs in 0,1 normaler HCl nach 2h betrug unter 20%. Nach Umpufferung auf pH 6,8 wurden 87% nach 10 H freigesetzt.

Nach 6 Monaten Lagerung bei 30°C/ 70 % rel. Feuchte waren die Zubereitungen immer noch amorph.

### Beispiel 16

600g Polymer 1, 1000g HPMCAS und 400g Fenofibrat (Schmelzpunkt 81°C) wurden in einen Turbulamischbehälter eingewogen und 10 Minuten im Turbulamischer T10B gemischt.

Die Mischung wurde unter folgenden Bedingungen extrudiert:
- Zonentemperatur 1. Zylinder: 20°C; 2. Zylinder: 40°C
- Zonentemperatur ab dem 3. Zylinder: 120°C
- Schneckendrehzahl 100 UpM
- Durchsatz:800g/h
- Düsendurchmesser 3mm

Die festen Lösungen wurden mit XRD und per DSC untersucht und als amorph befunden. Die Freisetzung des Wirkstoffs in 0,1 normaler HCl nach 2h betrug unter 10%, nach Umpufferung auf pH 6,8 wurden 100% freigesetzt

### Beispiel 17

Das Extrudat aus Beispiel 7 wurde mit einer Luftstrahlmühle auf eine Teilchengröße kleiner 25µm zerkleinert. Die Freisetzung des Wirkstoffs betrug in 0,1 normaler HCl nach 1 h 82%.

### Beispiel 18

Das Extrudat aus Beispiel 15 wurde mit einer Luftstrahlmühle auf eine Teilchengröße kleiner 20µm zerkleinert. Die Freisetzung des Wirkstoffs in 0,1 normaler HCl betrug nach 1 h 79%.

## Patentansprüche

1. Dosierungsformen, enthaltend Zubereitungen von in Wasser schwerlöslichen Wirkstoffen in einer Polymermatrix aus Polyether-Copolymeren, wobei die Polyether-Copolymere erhalten werden durch radikalisch initiierte Polymerisation einer Mischung aus 30 bis 80 Gew.-% N-Vinyllactam,10 bis 50 Gew.-% Vinylacetat und 10 bis 50 Gew.-% eines Polyethers, und aus mindestens einem in Wasser schwerlöslichen Polymeren, wobei "in Wasser schwer löslich" bedeutet, dass für eine Lösung des Polymers in Wasser bei 20°C mindestens 100 bis 1000 g Wasser pro g Polymer benötigt werden, in denen der in Wasser schwerlösliche Wirkstoff amorph in der Polymermatrix vorliegt.

2. Dosierungsformen nach Anspruch 1, enthaltend als in Wasser schwerlösliche Polymere solche Polymere, deren Löslichkeit in Wasser pH-Wert abhängig ist.

3. Dosierungsformen nach Anspruch 1 oder 2, enthaltend in Wasser schwerlösliche Polymere auf Basis von Acrylsäure oder Methacrylsäure oder deren Estern oder Mischungen der genannten Monomere.

4. Dosierungsformen nach Anspruch 1, enthaltend als in Wasser schwerlösliche Polymere Homo- und Copolymere des Vinylacetats.

5. Dosierungsformen nach Anspruch 1, enthaltend als in Wasser schwerlösliche Polymere Ethylcellulosen.

6. Dosierungsformen nach einem der Ansprüche 1 bis 5 , in denen das Verhältnis des Polyether-Copolymers zu dem in Wasser schwerlöslichen Polymer zwischen 99 :1 und 10 : 90 beträgt.

7. Dosierungsformen nach einem der Ansprüche 1 bis 6, in denen das Verhältnis des Polyether-Copolymers zu dem in Wasser schwerlöslichen Polymer zwischen 90 :10 und 30 : 70 beträgt.

8. Dosierungsformen nach einem der Ansprüche 1 bis 7, in denen das Verhältnis des Polyether-Copolymers zu dem in Wasser schwerlöslichen Polymer zwischen 80 :20 und 40 : 60 beträgt.

9. Dosierungsformen nach einem der Ansprüche 1 bis 8, in denen die Polymermatrix einen weiteren Löslichkeitsvermittler enthält.

10. Verfahren zur Herstellung von Zubereitungen für Dosierungsformen von in Wasser schwerlöslichen Wirkstoffen gemäß einem der Ansprüche 1 bis 9, wobei die Wirkstoffe amorph in einer Polymermatrix auf Basis von Polyether-Copolymeren aus 30 bis 80 Gew.-% N-Vinyllactam,10 bis 50 Gew.-% Vinylacetat und 10 bis 50 Gew.-% eines Polyethers eingebettet vorliegen, **dadurch gekennzeichnet, dass** in die Polymermatrix neben dem Polyether-Copolymeren mindestens ein in Wasser schwerlöslichen Polymer eingearbeitet wird und die Polymere mit den in Wasser schwerlöslichen Wirkstoffen innig vermischt werden.

11. Verfahren nach Anspruch 10, **dadurch gekennzeichnet, dass** die Mischung aus Polymeren und Wirkstoffen über die Glasübergangstemperatur der Polyether-Copolymeren erwärmt wird.

12. Verfahren nach Anspruch 10 oder 11, **dadurch gekennzeichnet, dass** die Mischung aus Polymeren und Wirkstoffen in einem Extruder erfolgt.

13. Verfahren nach Anspruch 10, **dadurch gekennzeichnet, dass** die Mischung aus Polymeren und Wirkstoffen in organischer Lösung erfolgt und die organischen Lösungsmittel anschließend entfernt werden.

14. Verfahren nach Anspruch 13, **dadurch gekennzeichnet, dass** die organischen Lösungsmittel durch Sprühtrocknung entfernt werden.

## Claims

1. A dosage form comprising preparations of slightly water-soluble active substances in a polymer matrix of polyether copolymers, the polyether copolymers being obtained by free radical polymerization of a mixture of from 30 to 80% by weight of N-vinyllactam, from 10 to 50% by weight of vinyl acetate and from 10 to 50% by weight of a polyether, and of at least one slightly water-soluble polymer, "slightly water-soluble" meaning that, for a solution of the polymer in water at 20°C, from at least 100 to 1000 g of water are required per g of polymer, in which the slightly water-soluble active substance is present in amorphous form in the polymer matrix.

2. The dosage form according to claim 1, comprising, as slightly water-soluble polymers, those polymers whose solubility in water is pH-dependent.

3. The dosage form according to claim 1 or 2, comprising slightly water-soluble polymers, based on acrylic acid or methacrylic acid or esters thereof or mixtures of said monomers.

4. The dosage form according to claim 1, comprising homo- and copolymers of vinyl acetate as slightly water-soluble polymers.

5. The dosage form according to claim 1, comprising ethylcelluloses as slightly water-soluble polymers.

6. The dosage form according to any of claims 1 to 5, in which the ratio of the polyether copolymer to the slightly water-soluble polymer is from 99:1 to 10:90.

7. The dosage form according to any of claims 1 to 6, in which the ratio of the polyether copolymer to the slightly water-soluble polymer is from 90:10 to 30:70.

8. The dosage form according to any of claims 1 to 7, in which the ratio of the polyether copolymer to the slightly water-soluble polymer is from 80:20 to 40:60.

9. The dosage form according to any of claims 1 to 8, in which the polymer matrix comprises a further solubilizer.

10. A process for the production of preparations for dosage forms of slightly water-soluble active substances according to any of claims 1 to 9, the active substances being present in amorphous form embedded in a polymer matrix based on polyether copolymers of from 30 to 80% by weight of N-vinyllactam, from 10 to 50% by weight of vinyl acetate and from 10 to 50% by weight of a polyether, wherein, in addition to the polyether copolymer, at least one slightly water-soluble polymer is incorporated into the polymer matrix, and the polymers are thoroughly mixed with the slightly water-soluble active substances.

11. The process according to claim 10, wherein the mixture of polymers and active substances is heated above the glass transition temperature of the polyether copolymers.

12. The process according to claim 10 or 11, wherein the mixing of polymers and active substances is effected in an extruder.

13. The process according to claim 10, wherein the mixing of polymers and active substances is effected in organic solution, and the organic solvents are then removed.

14. The process according to claim 13, wherein the organic solvents are removed by spray drying.

## Revendications

1. Formes de dosage, contenant des compositions de substances actives peu solubles dans l'eau dans une matrice polymère en copolymères de polyéther, les copolymères de polyéther étant obtenus par polymérisation initiée par voie radicalaire d'un mélange constitué par 30 à 80% en poids de N-vinyllactame, 10 à 50% en poids d'acétate de vinyle et 10 à 50% en poids d'un polyéther, et en au moins un polymère peu soluble dans l'eau, "peu soluble dans l'eau" signifiant qu'il faut, pour une solution du polymère dans l'eau à 20°C, au moins 100 à 1000 g d'eau par g de polymère, dans lesquelles la substance active peu soluble dans l'eau se trouve sous forme amorphe dans la matrice polymère.

2. Formes de dosage selon la revendication 1, contenant, comme polymères peu solubles dans l'eau, des polymères dont la solubilité dans l'eau dépend du pH.

3. Formes de dosage selon la revendication 1 ou 2, contenant des polymères peu solubles dans l'eau à base d'acide acrylique ou d'acide méthacrylique ou leurs esters ou des mélanges des monomères mentionnés.

4. Formes de dosage selon la revendication 1, contenant, comme polymères peu solubles dans l'eau, des homopolymères ou des copolymères d'acétate de vinyle.

5. Formes de dosage selon la revendication 1, contenant, comme polymères peu solubles dans l'eau, des éthylcelluloses.

6. Formes de dosage selon l'une quelconque des revendications 1 à 5, dans lesquelles le rapport du copolymère de polyéther au polymère peu soluble dans l'eau est situé entre 99:1 et 10:90.

7. Formes de dosage selon l'une quelconque des revendications 1 à 6, dans lesquelles le rapport du copolymère de polyéther au polymère peu soluble dans l'eau est situé entre 90:10 et 30:70.

8. Formes de dosage selon l'une quelconque des revendications 1 à 7, dans lesquelles le rapport du copolymère de polyéther au polymère peu soluble dans l'eau est situé entre 80:20 et 40:60.

9. Formes de dosage selon l'une quelconque des revendications 1 à 8, dans lesquelles la matrice polymère contient un autre promoteur de solubilisation.

10. Procédé pour la préparation des compositions pour des formes de dosage de substances actives peu solubles dans l'eau selon l'une quelconque des revendications 1 à 9, les substances actives se trouvant incorporées sous forme amorphe dans une matrice polymère à base de copolymères de polyéthers constitués par 30 à 80% en poids de N-vinyllactame, 10 à 50% en poids d'acétate de vinyle et 10 à 50% en poids d'un polyéther, **caractérisé en ce qu'**on incorpore dans la matrice polymère, outre les copolymères de polyéther, au moins un polymère peu soluble dans l'eau et les polymères étant mélangés intimement avec les substances actives peu solubles dans l'eau.

11. Procédé selon la revendication 10, **caractérisé en ce que** le mélange de polymères et de substances actives est chauffé au-dessus de la température de transition vitreuse des copolymères de polyéther.

12. Procédé selon la revendication 10 ou 11, **caractérisé en ce que** le mélange de polymères et de substances actives a lieu dans une extrudeuse.

13. Procédé selon la revendication 10, **caractérisé en ce que** le mélange de polymère et de substances actives a lieu en solution organique et les solvants organiques sont ensuite éliminés.

14. Procédé selon la revendication 13, **caractérisé en ce que** les solvants organiques sont éliminés par séchage par pulvérisation.
